# EUROPEAN PATENT APPLICATION

(11) **EP 4 393 327 A1**
(43) Date of publication of application: **03.07.2024**
(21) Application number: 22891983.3
(22) Date of filing: 08.11.2022
(51) Int. Cl.: A24F 40/10, A24F 40/40

(54) **ELASTIC CONDUCTIVE ELEMENT, ATOMIZATION ASSEMBLY AND ASSEMBLY METHOD THEREFOR, AND ULTRASONIC ATOMIZER**

(30) Priority: 09.11.2021 CN 202111319770
(71) Applicant: Shenzhen First Union Technology Co., Ltd., Shenzhen, Guangdong 518000 (CN)
(72) Inventor: XIE, Baofeng, Shenzhen, Guangdong 518000 (CN); XU, Zhongli, Shenzhen, Guangdong 518000 (CN); LI, Yonghai, Shenzhen, Guangdong 518000 (CN)
(74) Representative: Jacob, Reuben Ellis
(86) International application number: PCT/CN2022/130685
(87) International publication number: WO 2023/083189

(57) **Abstract**

This application provides an elastic conductive element, an atomization assembly and an assembly method therefor, and an ultrasonic atomizer. The atomization assembly includes an ultrasonic atomization sheet provided with a first electrode and a second electrode, where the ultrasonic atomization sheet is used for ultrasonically atomizing a liquid matrix to form liquid mist; and an elastic conductive element, which is in elastic contact with the ultrasonic atomization sheet to support the ultrasonic atomization sheet, where one area of the elastic conductive element is directly or indirectly electrically connected to the first electrode, and the other area of the elastic conductive element is directly or indirectly electrically connected to the second electrode, to form a loop among the first electrode, the elastic conductive element, and the second electrode. According to this application, energy stored by the ultrasonic atomization sheet after being powered on and then powered off is consumed and vibration transmission of the ultrasonic atomization sheet is reduced through the elastic conductive element, so that a resistance board does not need to be additionally provided, thereby reducing parts and costs of the atomization assembly, and saving a structural space of the atomization assembly.

## Description

### CROSS-REFERENCE TO RELATED APPLICATIONS

This application claims priority to Chinese Patent Application No. 202111319770.9, entitled "ELASTIC CONDUCTIVE ELEMENT, ATOMIZATION ASSEMBLY AND ASSEMBLY METHOD THEREFOR, AND ULTRASONIC ATOMIZER" and filed with the China National Intellectual Property Administration on November 09, 2021, which is incorporated herein by reference in its entirety.

### TECHNICAL FIELD

Embodiments of this application relate to the field of atomization technologies, and in particular, to an elastic conductive element, an atomization assembly and an assembly method therefor, and an ultrasonic atomizer.

### BACKGROUND

An ultrasonic atomizer includes an ultrasonic atomization sheet. The ultrasonic atomization sheet is provided with micropores. When the ultrasonic atomization sheet generates high-frequency vibration, a liquid matrix in the micropores can be atomized to form liquid mist. The liquid mist is squirted from the micropores to be inhaled by a user.

A resistance board is connected between an electrode on an upper surface and an electrode on a lower surface of the existing ultrasonic atomization sheet, so that after the ultrasonic atomizer is powered off, the ultrasonic atomization sheet and a resistor on the resistance board form a closed circuit, which can consume, after being powered on and then powered off, energy stored in the ultrasonic atomization sheet itself, to avoid a case that an instantaneous high voltage is released to burn out other electronic components after the ultrasonic atomization sheet is powered on again, to ensure that the ultrasonic atomization sheet can work normally after being powered on again.

The existence of the resistance board leads to many parts, many processes, and high costs of the ultrasonic atomizer, and the resistance board is fixedly and conductively connected to the electrode on the upper surface and the electrode on the lower surface of the ultrasonic atomization sheet by screws or solder, and is prone to poor soldering and other infirm fixation problems.

### SUMMARY

This application provides an elastic conductive element, an atomization assembly and an assembly method therefor, an ultrasonic atomizer, and an ultrasonic atomization apparatus, aiming to resolve problems of many parts and high costs of the existing ultrasonic atomizer.

An aspect of this application provides an atomization assembly, including:
an ultrasonic atomization sheet provided with a first electrode and a second electrode, where the ultrasonic atomization sheet is used for ultrasonically atomizing a liquid matrix to form liquid mist; and
an elastic conductive element, which is in elastic contact with the ultrasonic atomization sheet to support the ultrasonic atomization sheet, where
one area of the elastic conductive element is directly or indirectly electrically connected to the first electrode, and the other area of the elastic conductive element is directly or indirectly electrically connected to the second electrode, to form a loop among the first electrode, the elastic conductive element, and the second electrode.

Another aspect of this application provides an ultrasonic atomizer, including a liquid storage cavity for storing a liquid matrix, and the foregoing atomization assembly.

Another aspect of this application further provides an assembly method for an atomization assembly. The assembly method includes:
providing a conductive tube, where a first end of the conductive tube is provided with a first extending portion extending radially toward a center of the conductive tube;
mounting an ultrasonic atomization sheet in the conductive tube, where a first electrode of the ultrasonic atomization sheet abuts against the first extending portion; and
mounting a second electrical connection member and an elastic conductive element in the conductive tube, where the second electrical connection member abuts against a second electrode of the ultrasonic atomization sheet, and the elastic conductive element abuts against the ultrasonic atomization sheet.

Another aspect of this application further provides an assembly method for an atomization assembly. The assembly method includes:
providing a conductive tube, where a second end of the conductive tube is provided with a second extending portion extending radially toward a center of the conductive tube;
mounting a second electrical connection member and an elastic conductive element in the conductive tube, where the elastic conductive element abuts against the second extending portion, and the elastic conductive element spaces the conduit tube apart from the second electrical connection member;
mounting an ultrasonic atomization sheet in the conductive tube, where a first electrode of the ultrasonic atomization sheet abuts against the second electrical connection member; and
performing a bending operation on the second end of the conductive tube, to cause the first end to form a first extending portion extending radially toward the center of the conductive tube, where the first extending portion is connected to the first electrode of the ultrasonic atomization sheet.

Another aspect of this application further provides an elastic conductive element, including: a cylindrical body, where the cylindrical body includes a proximal end, a distal end, and a hollow part extending from the proximal end to the distal end;
the proximal end is at least partially used for coming into elastic contact with an ultrasonic atomization sheet to support the ultrasonic atomization sheet; the hollow part is used for accommodating a second electrical connection member, to fix the second electrical connection member, and an inner wall of the hollow part is at least partially in contact with the second electrical connection member to form electrical connection; and an outer wall of the cylindrical body is in contact with a first electrical connection member, to form electrical connection.

According to the elastic conductive element, the atomization assembly and the assembly method therefor, the ultrasonic atomizer, and the ultrasonic atomization apparatus provided in this application, energy stored by the ultrasonic atomization sheet after being powered on and then powered off is consumed and vibration transmission of the ultrasonic atomization sheet is reduced through the elastic conductive element, so that a resistance board does not need to be additionally provided, thereby reducing parts and costs of the atomization assembly, and saving a structural space of the atomization assembly.

### BRIEF DESCRIPTION OF THE DRAWINGS

One or more embodiments are exemplarily described with reference to the corresponding figures in the accompanying drawings, and the descriptions are not to be construed as limiting the embodiments. Elements in the accompanying drawings that have same reference numerals are represented as similar elements, and unless otherwise particularly stated, the figures in the accompanying drawings are not drawn to scale.
FIG. 1 is a schematic diagram of an ultrasonic atomization apparatus according to an implementation of this application;
FIG. 2 is a schematic diagram of another ultrasonic atomization apparatus according to an implementation of this application;
FIG. 3 is a schematic diagram of an atomization assembly according to an implementation of this application;
FIG. 4 is a schematic cross-sectional view of an atomization assembly according to an implementation of this application;
FIG. 5 is a schematic exploded view of an atomization assembly according to an implementation of this application;
FIG. 6 is a schematic diagram of an ultrasonic atomization sheet according to an implementation of this application;
FIG. 7 is a schematic diagram of an ultrasonic atomization sheet according to an implementation of this application from another perspective; and
FIG. 8 is a schematic diagram of an elastic conductive element according to an implementation of this application from another perspective.

### DETAILED DESCRIPTION

For ease of understanding this application, this application is described in more detail below with reference to the accompanying drawings and specific embodiments. It should be noted that, when an element is expressed as "being fixed to" another element, the element may be directly on the another element, or one or more intermediate elements may exist between the element and the another element. When an element is expressed as "being connected to" another element, the element may be directly connected to the another element, or one or more intermediate elements may exist between the element and the another element. The terms "upper", "lower", "left", "right", "inner", "outer", and similar expressions used in this specification are merely used for an illustrative purpose.

Unless otherwise defined, meanings of all technical and scientific terms used in this specification are the same as that usually understood by a person skilled in the art to which this application belongs. The terms used in this specification of this application are merely intended to describe objectives of the specific implementations, and are not intended to limit this application. The term "and/or" used in this specification includes any or all combinations of one or more related listed items.

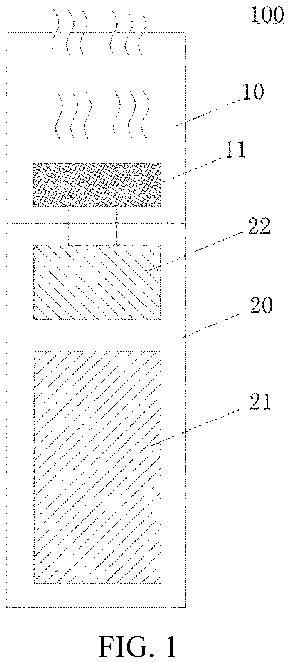

FIG. 1 is a schematic diagram of an ultrasonic atomization apparatus according to an implementation of this application.

As shown in FIG. 1, the ultrasonic atomization apparatus 100 includes an ultrasonic atomizer 10 and a power supply assembly 20, and the ultrasonic atomizer 10 and the power supply assembly 20 are non-detachable.

The ultrasonic atomizer 10 includes a liquid storage cavity (not shown in the accompanying drawing) for storing a liquid matrix, and an atomization assembly 11, and the atomization assembly 11 generates high-frequency oscillation under the action of power provided by the power supply assembly 20, so that the liquid matrix is atomized into liquid mist.

The power supply assembly 20 includes a cell 21 and a circuit 22.

The cell 21 supplies power for operating the ultrasonic atomization apparatus 100. The cell 21 may be a rechargeable cell or a disposable cell.

The circuit 22 may control overall operations of the ultrasonic atomization apparatus 100. The circuit 22 not only controls operations of the cell 21 and the atomization assembly 11, but also controls operations of other components in the ultrasonic atomization apparatus 100.

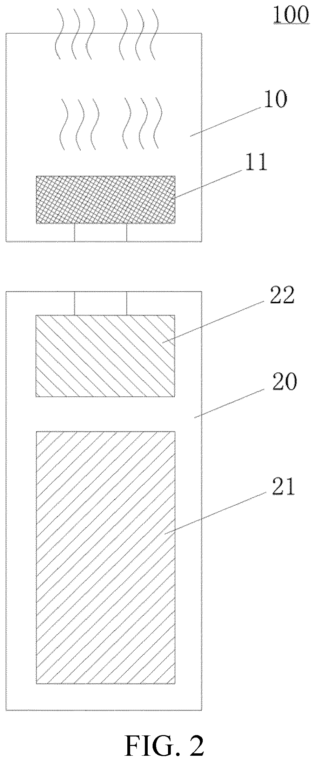

FIG. 2 is a schematic diagram of another ultrasonic atomization apparatus according to an implementation of this application. Different from the example in FIG. 1, an ultrasonic atomizer 10 is detachably connected to a power supply assembly 20.

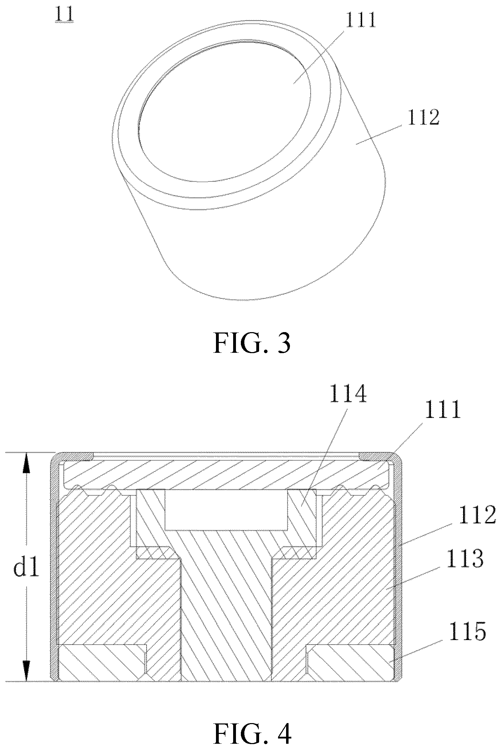

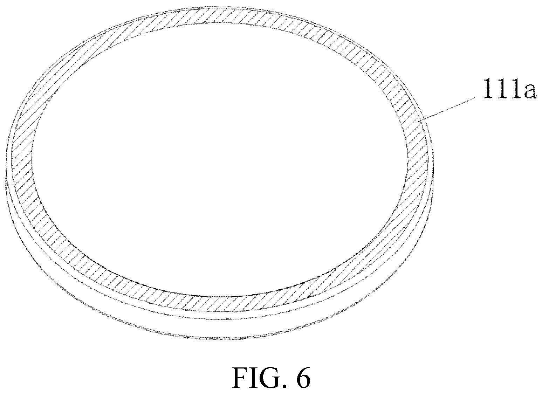

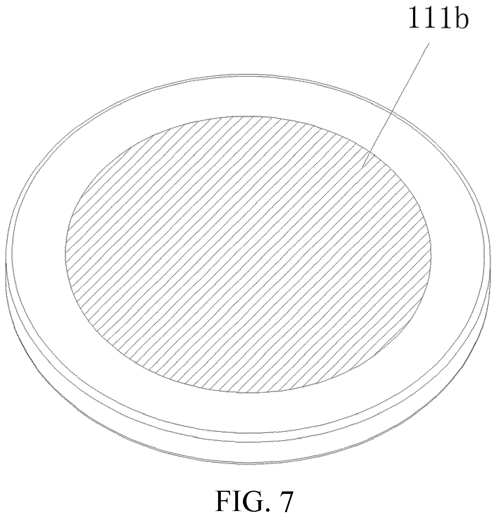

As shown in FIG. 3 to FIG. 7, an implementation of this application provides an atomization assembly 11, including an ultrasonic atomization sheet 111, a first electrical connection member (112, 115), an elastic conductive element 113, and a second electrical connection member 114.

In this example, the ultrasonic atomization sheet 111 includes a piezoelectric ceramic matrix, and the piezoelectric ceramic matrix is roughly circular, and has an upper surface on which a first electrode 11 1a is formed and a lower surface on which a second electrode 111b is formed.

The first electrical connection member (112, 115) includes a conductive tube 112 and a coupling member 115. The ultrasonic atomization sheet 111, the elastic conductive element 113, and the second electrical connection member 114 are each arranged in the conductive tube 112. Specifically, the ultrasonic atomization sheet 111 is horizontally arranged close to an upper end (a first end) of the conductive tube 112, the elastic conductive element 113 and the second electrical connection member 114 are arranged between the ultrasonic atomization sheet 111 and a lower end (a second end) of the conductive tube 112, and the conductive tube 112 is spaced apart from the second electrical connection member 114 through the elastic conductive element 113.

One area of the elastic conductive element 113 is directly or indirectly electrically connected to the first electrode 111a of the ultrasonic atomization sheet 111, and the other area of the elastic conductive element 113 is directly or indirectly electrically connected to the second electrode 111b, to form a loop among the first electrode 111a, the elastic conductive element 113, and the second electrode 111b. It should be noted that the one area and the other area of the elastic conductive element 113 may be any two areas or parts in the elastic conductive element 113, as long as a case that the first electrode 111a is directly in contact with the second electrode 111b to cause short circuit is avoided.

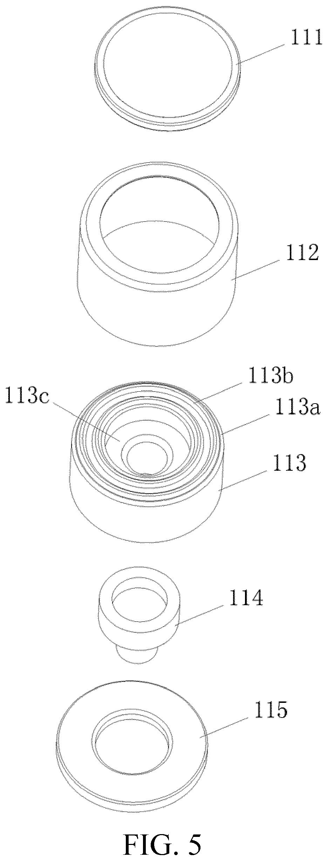

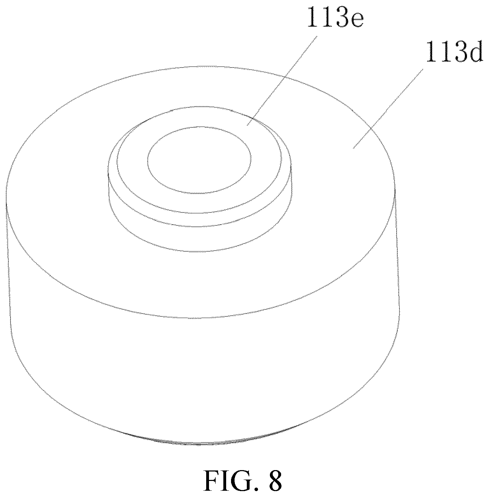

In a preferred implementation, as shown in FIG. 5 and FIG. 8, the elastic conductive element 113 is constructed into a cylindrical structural, that is, includes a cylindrical body, an upper end 113a (proximal end) of the cylindrical body is provided with a plurality of protruding portions 113b, the protruding portions 113b are ring-shaped, and the plurality of ring-shaped protruding portions 113b are arranged along the radial direction. A lower end 113d (distal end) of the cylindrical body is provided with a part extending axially downward, to form a boss 113e. A hollow part of the cylindrical body forms an accommodating chamber 113c, and the accommodating chamber 113c extends from the upper end 113a to the lower end 113d; and the accommodating chamber 113c matches the second electrical connection member 114 in shape, and is used for accommodating the second electrical connection member 114, to fix the second electrical connection member 114. The protruding portion 113b abuts against a lower surface of the ultrasonic atomization sheet 111, to support the ultrasonic atomization sheet 111. A sidewall of the elastic conductive element 113 at least partially abuts against an inner wall of the conductive tube 112, to form electrical connection.

The elastic conductive element 113 is made of a mixture of an elastic material and metal particles, and the elastic material may be silicone, soft rubber, or the like. In this way, the elastic conductive element 113 itself has specific resistance, whose resistance value is greater than 500 KS2; or is greater than 800 KS2; or is greater than 1 MΩ; or is greater than 1 MΩ and less than 2 MS2. The elastic conductive element 113 reduces vibration transmission of the ultrasonic atomization sheet 111 through its own elasticity in an aspect; and maintains contact with both the first electrical connection member and the second electrical connection member 114 to form electrical connection in another aspect, thereby forming a loop to consume energy stored in the ultrasonic atomization sheet 111 itself after being powered on and then powered off, to ensure that the ultrasonic atomization sheet 111 can work normally after being powered on again, and avoid a case that an instantaneous high voltage is released to burn out other electronic components after the ultrasonic atomization sheet 111 is powered on again. Because the resistance of the elastic conductive element 113 itself is large, while the resistance of the ultrasonic atomization sheet 111 is small, the elastic conductive element 113 consumes very little energy when the ultrasonic wave atomization 111 works. It may be understood that the elastic conductive element 113 further has a sealing effect, and can prevent the liquid matrix from leaking or flowing toward the lower end 113d in the cylindrical body because of maintaining contact with the lower surface of the ultrasonic atomization sheet 111 and the inner wall of the conductive tube 112.

In this example, because the elastic conductive element 113 is adopted, and no resistance board is additionally arranged, the distance d1 between the upper end and the lower end of the conductive tube 112 is much less than that in the existing solution. Usually, d1 ranges from 5 mm to 6 mm; or ranges from 5.2 mm to 6 mm; or ranges from 5.4 mm to 6 mm; or ranges from 5.5 mm to 6 mm.

The lower surface of the ultrasonic atomization sheet 111 faces the second electrical connection member 114. In this way, one end of the second electrical connection member 114 abuts against the second electrode 111b formed on the lower surface of the ultrasonic atomization sheet 111 to maintain contact thereby forming electrical connection, and the other end of the second electrical connection member 114 extends toward the lower end of the conductive tube 112. The upper end of the conductive tube 112 is provided with an extending portion (not shown in the accompanying drawing) that extends radially, and the extending portion abuts against the first electrode 111a formed on the upper surface of the ultrasonic atomization sheet 111 to maintain contact thereby forming electrical connection.

It should be noted that the first electrode 111a and the second electrode 111b on the ultrasonic atomization sheet 111 are not limited to the situation in the accompanying drawing. In another example, the first electrode 111a and the second electrode 111b may be arranged on two sides of the ultrasonic atomization sheet 111 separately, or may be arranged on one side, or may be arranged on the same surface of the ultrasonic atomization sheet 111 (for example, both arranged on the lower surface of the ultrasonic atomization sheet 111). As positions of the first electrode 111a and the second electrode 111b change, structures of the elastic conductive element 113, the first electrical connection member (112, 115), and the second electrical connection member 114 may also correspondingly change. For example, the elastic conductive element 113 may be electrically connected to the first electrode 111a and the second electrode 111b on one side of the ultrasonic atomization sheet 111; or the elastic conductive element 113 may be electrically connected to the first electrode 111a on one side and electrically connected to the second electrode 111b on the other side in a manner of wrapping the ultrasonic atomization sheet 111.

The coupling member 115 is arranged close to the lower end of the conductive tube 112 and is maintained on the boss 113e. The coupling member 115 is ring-shaped and may be socketed on the boss 113e, and the coupling member 115 maintains contact with the conductive tube 112 to form electrical connection. In this way, when the atomization assembly 11 is coupled to the cell 21, a lead may be advantageously soldered to the coupling member 115 and the other end of the second electrical connection member 114.

In the examples of FIG. 3 to FIG. 8, the first electrical connection member (112, 115) includes the conductive tube 112 and the coupling member 115, that is, the first electrical connection member is formed by two or more separate members. Different from the examples of FIG. 3 to FIG. 8, in another example, the first electrical connection member is realized by one structural member or an integrally formed structural member, which is also feasible.

In the examples of FIG. 3 to FIG. 8, the elastic conductive element 113 maintains contact with the first electrical connection member to form electrical connection, and the elastic conductive element 113 maintains contact with the second electrical connection member 114 to form electrical connection, that is, indirectly electrically connected to the first electrode 111a through the first electrical connection member, and indirectly electrically connected to the second electrode 111b through the second electrical connection member 114. Different from the examples of FIG. 3 to FIG. 8, in another example, the elastic conductive element 113 does not maintain contact with the first electrical connection member, and the elastic conductive element 113 does not maintain contact with the second electrical connection member 114, which is also feasible. Specifically, the elastic conductive element 113 directly maintains contact with the first electrode 111a thereby forming electrical connection, and the elastic conductive element 113 directly maintains contact with the second electrode 111b thereby forming electrical connection. Further, the elastic conductive element 113 maintains contact with both the first electrode 111a and the first electrical connection member thereby forming electrical connection, and the elastic conductive element 113 maintains contact with both the second electrode 111b and the second electrical connection member 114 thereby forming electrical connection, which is also feasible.

In the examples of FIG. 3 to FIG. 8, the atomization assembly 11 formed by the ultrasonic atomization sheet 111, the first electrical connection member (112, 115), the elastic conductive element 113, and the second electrical connection member 114 is arranged in the ultrasonic atomizer 10 (detachable or non-detachable from the power supply assembly 20). Different from the examples of FIG. 3 to FIG. 8, in another example, the atomization assembly 11 formed by the ultrasonic atomization sheet 111 and the elastic conductive element 113 may be arranged in the ultrasonic atomizer 10 (detachable or non-detachable from the power supply assembly 20); and the first electrical connection member (112, 115) and the second electrical connection member 114 are arranged in the power supply assembly 20, which is also feasible. In this case, the first electrical connection member (112, 115) and the second electrical connection member 114 may be electrically connected to the electrodes in the ultrasonic atomization sheet directly or indirectly.

Another implementation of this application further provides an assembly method for an atomization assembly. The assembly method includes:
Step S11. Provide a conductive tube, where a first end of the conductive tube is provided with a first extending portion extending radially toward a center of the conductive tube.
Step S12. Mount an ultrasonic atomization sheet in the conductive tube, where a first electrode of the ultrasonic atomization sheet abuts against the first extending portion.
Step S13. Mount a second electrical connection member and an elastic conductive element in the conductive tube, where the second electrical connection member abuts against a second electrode of the ultrasonic atomization sheet, and the elastic conductive element abuts against the ultrasonic atomization sheet.

In this step, the second electrical connection member may be first mounted in the conductive tube, and then the elastic conductive element is mounted in the conductive tube; or the second electrical connection member and the elastic conductive element that are assembled are mounted in the conductive tube together.

In an example, after the mounting a second electrical connection member and an elastic conductive element in the conductive tube, the assembly method further includes:
mounting a coupling member in the conductive tube, where the coupling member separately abuts against an inner wall of the conductive tube and the elastic conductive element.

In an example, after the mounting a second electrical connection member and an elastic conductive element in the conductive tube, the assembly method further includes:
bending the second end of the conductive tube, to cause the second end to form a second extending portion extending radially toward the center of the conductive tube.

In this example, the second extending portion formed by bending the second end of the conductive tube is functionally similar to the coupling member 115 in the examples of FIG. 3 to FIG. 8.

Another implementation of this application further provides an assembly method for an atomization assembly. The assembly method includes:
Step S21. Provide a conductive tube, where a second end of the conductive tube is provided with a second extending portion extending radially toward a center of the conductive tube.

In this step, the second extending portion of the second end is functionally similar to the coupling member 115 in the examples of FIG. 3 to FIG. 8.

Step S22. Mount a second electrical connection member and an elastic conductive element in the conductive tube, where the elastic conductive element abuts against the second extending portion, and the elastic conductive element spaces the conduit tube apart from the second electrical connection member.

In this step, the second electrical connection member may be first mounted in the conductive tube, and then the elastic conductive element is mounted in the conductive tube; or the second electrical connection member and the elastic conductive element that are assembled are mounted in the conductive tube together.

Step S23. Mount an ultrasonic atomization sheet in the conductive tube, where a first electrode of the ultrasonic atomization sheet abuts against the second electrical connection member.

Step S24. Perform a bending operation on the second end of the conductive tube, to cause the first end to form a first extending portion extending radially toward the center of the conductive tube, where the first extending portion is connected to the first electrode of the ultrasonic atomization sheet.

It should be noted that the specification of this application and the accompanying drawings thereof illustrate preferred embodiments of this application. However, this application may be implemented in various different forms, and is not limited to the embodiments described in this specification. These embodiments are not intended to be an additional limitation on the content of this application, and are described for the purpose of providing a more thorough and comprehensive understanding of the content disclosed in this application. Moreover, the foregoing technical features are further combined to form various embodiments not listed above, and all such embodiments shall be construed as falling within the scope of this application. Further, a person of ordinary skill in the art may make improvements or modifications according to the foregoing description, and all the improvements and modifications shall fall within the protection scope of the attached claims of this application.

## Claims

1. An atomization assembly, comprising:
an ultrasonic atomization sheet provided with a first electrode and a second electrode, wherein the ultrasonic atomization sheet is used for ultrasonically atomizing a liquid matrix to form liquid mist; and
an elastic conductive element, which is in elastic contact with the ultrasonic atomization sheet to support the ultrasonic atomization sheet, wherein
one area of the elastic conductive element is directly or indirectly electrically connected to the first electrode, and the other area of the elastic conductive element is directly or indirectly electrically connected to the second electrode, to form a loop among the first electrode, the elastic conductive element, and the second electrode.

2. The atomization assembly according to claim 1, wherein a resistance value of the elastic conductive element is greater than 500 KS2; or a resistance value of the elastic conductive element is greater than 800 KS2; or a resistance value of the elastic conductive element is greater than 1 MΩ.

3. The atomization assembly according to claim 1, wherein the atomization assembly further comprises a first electrical connection member in contact with the first electrode, and/or a second electrical connection member in contact with the second electrode;
the elastic conductive element is in contact with the first electrical connection member, to be indirectly electrically connected to the first electrode through the first electrical connection member; and
the elastic conductive element is in contact with the second electrical connection member, to be indirectly electrically connected to the second electrode through the second electrical connection member.

4. The atomization assembly according to claim 3, wherein the first electrical connection member comprises a conductive tube, and the conductive tube is electrically connected to the first electrode; and
the ultrasonic atomization sheet, the second electrical connection member, and the elastic conductive element are each arranged in the conductive tube, and the conductive tube is spaced apart from the second electrical connection member through the elastic conductive element.

5. The atomization assembly according to claim 4, wherein the conductive tube is provided with a first end and a second end opposite to the first end; and
a distance from the first end to the second end ranges from 5 mm to 6 mm; or ranges from 5.2 mm to 6 mm; or ranges from 5.4 mm to 6 mm; or ranges from 5.5 mm to 6 mm.

6. The atomization assembly according to claim 4, wherein the conductive tube is provided with a first end and a second end opposite to the first end; and
the ultrasonic atomization sheet is provided with a first surface used for arranging the first electrode and a second surface used for arranging the second electrode;
the first end is provided with an extending portion extending toward a radial direction in the conductive tube, and the extending portion is in contact with the first surface of the ultrasonic atomization sheet and abuts against the first electrode to form electrical connection; and
the second electrical connection member is in contact with the second surface of the ultrasonic atomization sheet and abuts against the second electrode to form electrical connection.

7. The atomization assembly according to claim 4, wherein the elastic conductive element is constructed into a cylindrical body; a hollow part of the cylindrical body is used for accommodating the second electrical connection member, to fix the second electrical connection member; and an inner wall of the hollow part is at least partially in contact with the second electrical connection member, and an outer wall of the cylindrical body is at least partially in contact with an inner wall of the conductive tube.

8. The atomization assembly according to claim 4, wherein the conductive tube is provided with a first end and a second end opposite to the first end; and
the first electrical connection member further comprises a coupling member arranged close to the second end, and the coupling member maintains contact with the conductive tube to form electrical connection.

9. The atomization assembly according to claim 8, wherein the elastic conductive element is provided with a boss close to the second end; and the coupling member is ring-shaped, and the coupling member is sleeved on the boss and is spaced apart from the second connection member through the boss.

10. The atomization assembly according to claim 1, wherein the elastic conductive element is made of at least a mixture of an elastic material and metal particles.

11. An ultrasonic atomizer, comprising a liquid storage cavity for storing a liquid matrix, and the atomization assembly according to any one of claims 1 to 10.

12. An assembly method for an atomization assembly, wherein the assembly method comprises:
providing a conductive tube, wherein a first end of the conductive tube is provided with a first extending portion extending radially toward a center of the conductive tube;
mounting an ultrasonic atomization sheet in the conductive tube, wherein a first electrode of the ultrasonic atomization sheet abuts against the first extending portion; and
mounting a second electrical connection member and an elastic conductive element in the conductive tube, wherein the second electrical connection member abuts against a second electrode of the ultrasonic atomization sheet, and the elastic conductive element abuts against the ultrasonic atomization sheet.

13. The assembly method according to claim 12, wherein after the mounting a second electrical connection member and an elastic conductive element in the conductive tube, the assembly method further comprises:
mounting a coupling member in the conductive tube, wherein the coupling member separately abuts against an inner wall of the conductive tube and the elastic conductive element.

14. The assembly method according to claim 12, wherein the conductive tube is provided with a second end opposite to the first end; and after the mounting a second electrical connection member and an elastic conductive element in the conductive tube, the assembly method further comprises:
bending the second end of the conductive tube, to cause the second end to form a second extending portion extending radially toward the center of the conductive tube.

15. An assembly method for an atomization assembly, wherein the assembly method comprises:
providing a conductive tube, wherein a second end of the conductive tube is provided with a second extending portion extending radially toward a center of the conductive tube;
mounting a second electrical connection member and an elastic conductive element in the conductive tube, wherein the elastic conductive element abuts against the second extending portion, and the elastic conductive element spaces the conduit tube apart from the second electrical connection member;
mounting an ultrasonic atomization sheet in the conductive tube, wherein a first electrode of the ultrasonic atomization sheet abuts against the second electrical connection member; and
performing a bending operation on the second end of the conductive tube, to cause the first end to form a first extending portion extending radially toward the center of the conductive tube, wherein the first extending portion is connected to the first electrode of the ultrasonic atomization sheet.

16. An elastic conductive element, comprising: a cylindrical body, wherein the cylindrical body comprises a proximal end, a distal end, and a hollow part extending from the proximal end to the distal end;
the proximal end is at least partially used for coming into elastic contact with an ultrasonic atomization sheet to support the ultrasonic atomization sheet; the hollow part is used for accommodating a second electrical connection member, to fix the second electrical connection member, and an inner wall of the hollow part is at least partially in contact with the second electrical connection member to form electrical connection; and an outer wall of the cylindrical body is at least partially in contact with a first electrical connection member, to form electrical connection.
